# EUROPEAN PATENT APPLICATION

(11) **EP 2 335 710 A1**
(43) Date of publication of application: **22.06.2011**
(21) Application number: 09811294.9
(22) Date of filing: 03.09.2009
(51) Int. Cl.: A61K 33/00, A01N 1/02, A61P 9/10

(54) **ELECTRON IRRADIATED PHYSIOLOGICALLY ISOTONIC SOLUTION, ELECTRON IRRADIATION DEVICE FOR ELECTRON IRRADIATED PHYSIOLOGICALLY ISOTONIC SOLUTION, AND ORGAN STORAGE CONTAINER**

(30) Priority: 03.09.2008 US 190827
(71) Applicant: Cambwick Healthcare K.K., Tokyo 105-0022 (JP)
(72) Inventor: HIRASAWA, Keisuke, Hachioji-shi Tokyo 193-0832 (JP); DOZEN, Masaharu, Tokyo 144-0056 (JP); TADA, Yuki, Tokyo 112-0002 (JP); OKIHARA, Daijiro, Hadano-shi Kanagawa 259-1302 (JP)
(74) Representative: Andres, Mark
(86) International application number: PCT/JP2009/004363
(87) International publication number: WO 2010/026764

(57) **Abstract**

An infusion solution bag 11 filled with a physiological saline and an infusion pump 12 are connected to an electron irradiation apparatus 13. The electron irradiation apparatus 13 is connected to a DC-type high voltage generator 2 connected with a DC power supply 1. Using the electron irradiation apparatus 13, an electron-irradiated physiological saline is prepared and administered intravenously to a human body 14. The electron irradiation apparatus 13 has a flow path 16 for the physiological saline inside the cylindrical column. In a first side of the column, a dielectric material 19 is closely attached to a cathode electrode 20. In a second side of the column, a conductive material pad 21 is closely attached to an anode electrode 22. Third and fourth sides between the electrodes 20 and 22 are composed of an insulation material 23 insulating each of the electrodes.

## Description

### TECHNICAL FIELD

The present invention relates to an electron-irradiated physiological isotonic solution and an electron irradiation apparatus for preparing the electron-irradiated physiological isotonic solution. Especially, the present invention relates to a technology to prepare the physiological isotonic solution having reducibility which is advantageous for anti-oxidative stress therapy, by irradiating an object to be irradiated, that is, a known physiological isotonic solution with discharged electrons. In the present invention, the isotonic solutions include physiological saline solution, Ringer's solution, organ preservation solution, and lactate solution that can also be used as injection and infusion solutions. The present invention also relates to an organ preservation container utilizing said electron irradiation apparatus.

### BACKGROUND ART

A living body has an advanced defense system against oxidative stresses induced by active oxygen that is produced during normal activity of the living body.

However, the anti-oxidative defense system maintained by the living body cannot sufficiently work against drastic and excessive oxidative stress observed in by brain and cardiac ischemia, which leads to severe tissue injuries accompanied by massive cell death and aftereffects.

Such tissue injuries are caused as follows. Firstly, arterial blockage, such as blood clots, etc. causes ischemic condition in the tissue. Then, when the ischemic condition is mediated by thrombolytic treatment, etc., a large amount of active oxygen species causing tissue injuries are produced by a drastic reperfusion of fresh blood. It is in general known as ischemia/reperfusion injury.

In addition, such ischemia/reperfusion injuries due to active oxygen species are also observed at the times of ischemic conditions during general surgical operations and organ preservation for transplantation. Thus, reduction of oxidative stress is a critical issue in the medical field.

As a conventional resolution for this problem, it has been proposed that various anti-oxidative substances of reduced form such as vitamins, glutathione, and active oxygen eliminators are administered from the exterior of the living body. However, the results have not been satisfactory yet. Thus, it is a very important issue in the medical field to provide a safe and new anti-oxidative substance and a therapy using thereof.

In order to solve the problems, the inventors focused on the oxidation and reduction theory suggesting that molecules deprived of electrons are oxidized and the molecules provided with electrons are reduced. That is, by irradiating an aqueous solution containing oxidized antioxidant with weak electrons, reduction of hydrogen ion as well as reduction of oxidized glutathione in the physiologic isotonic solution irradiated with electrons were observed. Therefore, it was found that the reducing ability can be induced in a solution by electron irradiation.

Moreover, as a result of intensive investigation, the inventors found out that physiological saline solution, irradiated with electrons, comes to possess the reducing ability in itself, and the solution is effective in alleviation of ischemic/reperfusion injuries, in which acute and a large amount of active oxygen species cause organ failures.

Incidentally, there exist Patent document 1 and Patent document 2, which describes a conventional technology that by electron irradiation, liquids such as water or aqueous solution are made to have functions that has not been existed before irradiation.

The invention disclosed in the Patent document 1 is to treat an aqueous solution containing germs and viruses harmful to the human body by using light, sound waves, and electron so as to produce active oxygen species, that is, oxidants, such as hydrogen peroxide and ozone, which detoxify the aqueous solution.

The invention disclosed in the Patent document 2 is to produce active oxygen species on the metal surface, such as hydrogen peroxide and superoxide, which are oxygen-derived products by means of electrons that is produced by light irradiation, and thereby preventing the metal from corrosion due to germs and fungi due to the sterilizing effects of those active oxygen species.
Patent document 1: Japanese translated version of PCT application Laid-open No. 2003-531875
Patent document 2: Japanese patent application Laid-open No. 2002-273238
Non-patent document 1: Fukuda K, Asoh S, Ishikawa M, Yamamoto Y, Ohsawa I, Ohta S. "Inhalation of hydrogen gas suppresses hepatic injury caused by ischemia/reperfusion through reducing oxidative stress", Biochem Biophys Res Commun. 2007 361(3) 670-4.

### DISCLOSURE OF THE INVENTION

However, the before-mentioned conventional technologies are methods utilizing powerful oxidizers, that is, active oxygen species produced by electrons, but not utilizing induction of reducing ability due to electron irradiation. Moreover, in the inventions of conventional technologies, a subject to be irradiated with electrons is an aqueous solution containing germs, virus, etc. harmful to the human body, or the purpose thereof is to prevent a metal from corrosion due to germs and fungi. It was not intended to use a physiological isotonic solution for the purpose of alleviating ischemic/reperfusion injuries. Therefore, such inventions of the conventional technologies cannot be applied to the physiological isotonic solution of the present invention or for the purpose of preparing such solution.

In particular, the physiological isotonic solution for the purpose of alleviating ischemic/reperfusion injuries needs to be administered to a patient continuously. However, Patent document 1 or Patent document 2 never discloses an electron irradiation apparatus which is suitable for preparing the physiological isotonic solution applicable to such requirements.

The present invention is made to solve the before-mentioned problems, and an object of the present invention is to provide an electron-irradiated physiological isotonic solution that is capable of alleviating clinically problematic ischemic/reperfusion cell injuries at brain infarction, myocardial infarction, and surgical operations.

Another object of the present invention is to provide an electron irradiation apparatus that is capable of continuously preparing said electron-irradiated physiological isotonic solution. Further object of the present invention is to provide an electron irradiation apparatus that can be used at bedside for infusing the electron-irradiated physiological isotonic solution having the reducing ability to a patient and that also can be used as an organ preservation container.

An electron-irradiated physiological isotonic solution according to the present invention is prepared by an irradiation method in which it is necessary to shift the oxidation reduction potential (ORP) to minus level accompanying with a rise of the pH value in the physiological isotonic solution after electron irradiation. Additionally, an injection solution and an infusion solution consisting of said electron-irradiated physiological isotonic solution used for alleviating ischemic/reperfusion injury are also other aspects of the present invention.

For the above purposes, it is necessary to prepare an electron-irradiated physiological isotonic solution, in which a decreased value of ORP is in a range of Δ30 mV to Δ608 mV or an increased value of pH is in a range of Δ0.36 to Δ4.47. This is because when the decreased value of ORP is smaller than Δ30mV or when the increased value of pH is smaller than Δ0.36, the physiological isotonic solution does not have functions as an injection solution or an infusion solution for alleviating the ischemic/reperfusion injuries. On the other hand, the electron-irradiated physiological isotonic solution of which decreased value of ORP is greater than Δ608mV (it should be less than Δ1000mV, preferably) is effective as injection solution, infusion solution, and organ reservation solution for the purpose of alleviating ischemic/reperfusion cell injuries and others; however, in order to obtain the decreased value of ORP greater than Δ608mV, it is necessary to take measures such as extending the length of the irradiation column of the currently used apparatus, which is complicated and difficult to be manufactured. Furthermore, since there is a limitation in the range of pH applicable for the medical use, it is reasonable to employ the solution having said producible pH range for medical application.

An electron irradiation apparatus for preparing the electron-irradiated physiological isotonic solution according to the present invention comprises:
a sealed column including a flow path for a physiological isotonic solution inside thereof, and also including an inlet and an outlet for the physiological isotonic solution communicating with the flow path at both ends thereof;
an electron discharging portion formed on a surface of the column by disposing a dielectric material and a cathode electrode closely-attached on an outside of the dielectric material;
an electron receiving portion formed on an opposite surface of the column to said surface provided with the cathode electrode by disposing a conductive material and an anode electrode closely attached on an outside of the conductive material; and
a high voltage generator for applying a DC voltage between the electrodes equipped with a power supply thereof.

In another aspect of the present invention, a bag for supplying a physiological isotonic solution to be irradiated with electrons is connected to the inlet of the electron irradiation apparatus, and a tube for supplying the electron-irradiated physiological isotonic solution as an infusion solution is connected to the outlet of the electron irradiation apparatus. Furthermore, in another aspect of the present invention, the electron irradiation apparatus for preparing the electron-irradiated physiological isotonic solution is combined with an organ preservation container.

The electron-irradiated physiological isotonic solution according to the present invention is capable of alleviating the drastic and excessive oxidative stress produced in ischemic/reperfusion injuries, thereby alleviating lethal injuries and aftereffects in brain infarction, myocardial infarction, etc.

By utilizing the electron irradiation apparatus for preparing the electron-irradiated physiological isotonic solution according to the present invention, it is achieved that the electron-irradiated physiological isotonic solution can be prepared at the bedside and continuously infused via the vein as an injection solution and an infusion solution, thereby alleviating acute oxidative stress induced by clinically problematic ischemic/reperfusion injuries in brain infarction, myocardial infarction and after surgical operation.

In application of the electron irradiation apparatus for preparing the electron-irradiated physiological isotonic solution according to the present invention into the organ preservation container, organs for transplantation placed inside the container can be preserved for a long time by electrically irradiating the organ preservation solution.

### BRIEF DESCRIPTION OF THE DRAWINGS

Fig. 1 is a layout diagram showing a first embodiment of the electron irradiation apparatus according to the present invention.
Fig. 2 is a graph showing the decreased value of ORP in the electron-irradiated physiological saline.
Fig. 3 is a graph showing the increased value of pH in the electron-irradiated physiological saline.
Fig. 4 is a graph showing conversion of oxidized glutathione into reduced glutathione in the electron-irradiated physiological saline.
Fig. 5 is a graph showing an example that the electron-irradiated physiological saline inhibits the increase of alanine aminotransferase (ALT) activity in a serum of liver ischemic/reperfusion injuries model.
Fig. 6 is a graph showing the inhibitory effects of the electron-irradiated physiological saline and the degassed electron-irradiated physiological saline against the increase of ALT activity in the serum of the liver ischemic/reperfusion injuries model.
Fig. 7 is a graph showing the inhibitory effects of the hydrogen-dissolved physiological saline and the degassed hydrogen-dissolved physiological saline against the increase of ALT activity in the serum of the liver ischemic/reperfusion injuries model.
Fig. 8 is a layout diagram showing the electron irradiation apparatus used in providing a patient with the electron-irradiated physiological saline in medical practice (a second embodiment according to the present invention).
Fig. 9 is an enlarged perspective view of the electron irradiation apparatus used in the second embodiment.
Fig. 10 is an enlarged perspective view showing a modified example of the electron irradiation apparatus used in the second embodiment.
Fig. 11 is an enlarged perspective view showing a modified example of the electron irradiation apparatus used in the second embodiment.
Fig. 12 is an enlarged perspective view showing a modified example of the electron irradiation apparatus used in the second embodiment.
Fig. 13 is a graph showing the effect of the distance between the both electrodes with respect to the reducing potency (ΔORP mV × volume mL).
Fig. 14 is a graph showing the decreased value of ORP in the electron-irradiated physiological saline prepared by the electron irradiation apparatus of the second embodiment.
Fig. 15 is a graph showing the increased value of pH in the electron-irradiated physiological saline prepared by the electron irradiation apparatus of the second embodiment.
Fig. 16 is a layout diagram of a third embodiment where the electron irradiation apparatus is connected to the organ preservation container.
Fig. 17 is a perspective view showing a fourth embodiment where the electron irradiation apparatus and the organ preservation container are integrally configured.

### EXPLANATION OF REFERENCE NUMERALS

- 1: DC power supply
- 2: DC-type high voltage generator
- 3: Negative output terminal of the DC-type high voltage generator
- 4: Needle electrode
- 5: Physiological saline
- 6: Acrylic cylinder
- 7: Filter
- 8: Rubber ring
- 9: Brass electrode
- 10: Positive output terminal of the DC-type high voltage generator
- 11: Infusion solution bag
- 12: Infusion pump
- 13: Electron irradiation apparatus
- 14: Human body
- 15: Inlet for the physiological isotonic solution
- 16: Flow path of the physiological isotonic solution
- 17: Outlet for the physiological isotonic solution
- 18: Lid
- 19: Dielectric material
- 20: Cathode electrode (Electron discharging portion)
- 21: Conductive material pad
- 22: Anode electrode (Electron receiving portion)
- 23: Insulation material
- 24: Organ preservation container
- 25: Organ preservation solution
- 26: Donor organ
- 27: Insulator
- 28: Refrigeration layer using ice

### BEST MODE FOR CARRYING OUT THE INVENTION

### (First Embodiment)

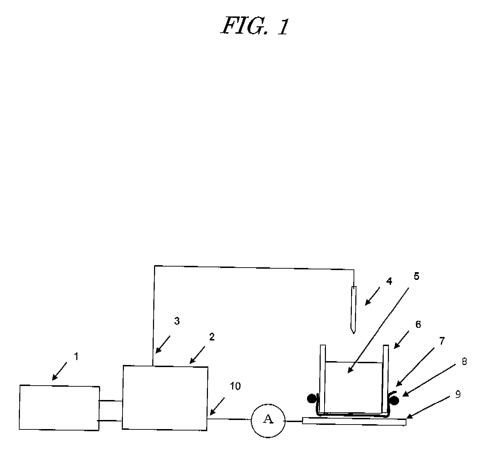

As shown in Fig. 1, a physiological saline 5 was filled into an acrylic cylinder 6 of which bottom side was covered by a filter 7 fixed with a rubber ring 8, and a needle electrode 4 was placed above the liquid level so that the needle never touches the saline. The needle electrode 4 was connected to a negative output terminal 3 of a DC-type high voltage generator 2 connected to a DC power supply 1. Below the acrylic cylinder 6, a brass electrode 9 was placed as a ground terminal, and the brass electrode 9 was connected to a positive output terminal 10 of the DC-type high voltage generator 2.

Using the DC-type high voltage generator 2, a high voltage of minus 5,000 volts producing a current of 10 micro amperes was applied between the needle electrode 4 and the brass electrode 9. The duration of application was 60 minutes. As a result, the physiological saline in the acrylic cylinder 6 was irradiated with electrons flowing between the needle electrode 4 and the brass electrode 9. Accordingly, the electron-irradiated physiological saline according to the first embodiment can be obtained.

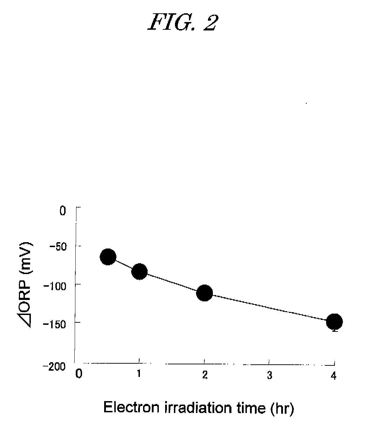

As shown in Fig. 2, the decreased value of ORP of thus obtained electron-irradiated physiological saline was about Δ80mV after the one-hour irradiation and it was time-dependent. Thus, the increase of reducing ability in the irradiated solution was observed.

In the generally used physiological saline, the ORP is around 400 to 500mV, however, in the electron-irradiated physiological saline of the first embodiment, it is confirmed that the ORP of about Δ80mV was decreased after the one-hour irradiation in comparison with the ORP of physiological saline before irradiation.

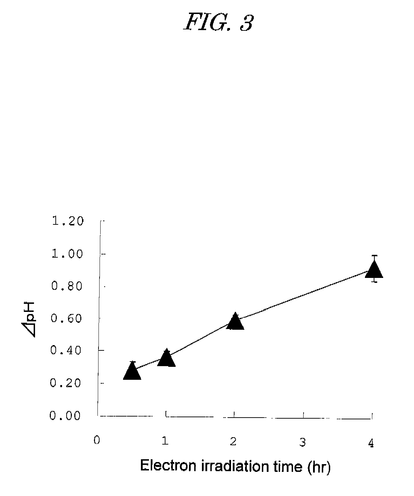

As shown in Fig. 3, the level of pH of the electron-irradiated physiological saline was increased by Δ0.36±0.04 from 6.55±0.06 after the one-hour irradiation and it was time-dependent. At this time, the current was 10 micro amperes and the voltage was minus 4,000 volts.

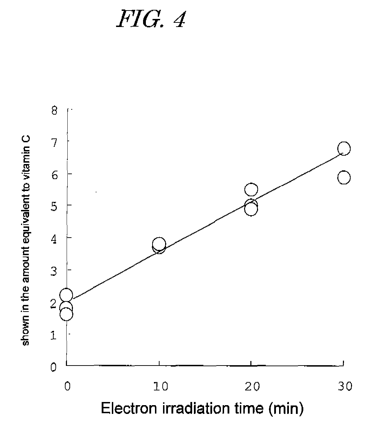

As shown in Fig. 4, similar electron irradiation was conducted to a physiological saline in which glutathione of the oxidized form was dissolved (6mg/mL). It was observed that glutathione of the reduced form was increased time-dependently.

Then, the inventors used a partial ischemic/reperfusion injuries model, in which ischemia was induced by clamping the rat liver artery and vein for 60 minutes and then reperfusion was performed by unclamping, then investigated the inhibitory effect of the electron-irradiated physiological saline on tissue injuries.

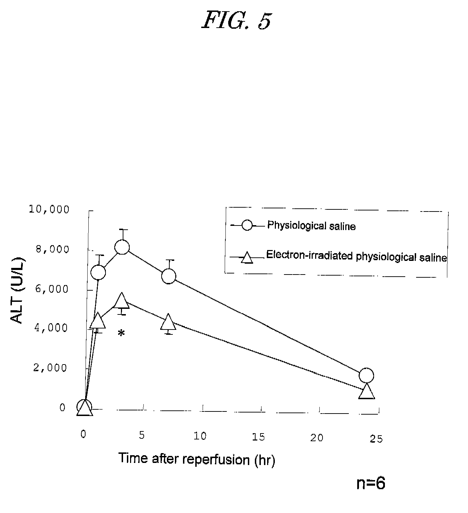

The electron-irradiated physiological saline was prepared by the method shown in the first embodiment, and 1.5mL of the saline was administered to the vein just after reperfusion. The results are shown in Fig. 5. The level of serum alanine aminotransferase activity (ALT level) after 3 hours from the reperfusion was 5,529±744 U/L. The inhibitory rate thereof was 32.4% in comparison with the group administered with the non-irradiated physiological saline, of which ALT value was 8,179±916 U/L.

These results show that the electron-irradiated physiological saline has an ability to alleviate tissue injuries induced by acute oxidative stress in ischemic/reperfusion injuries.

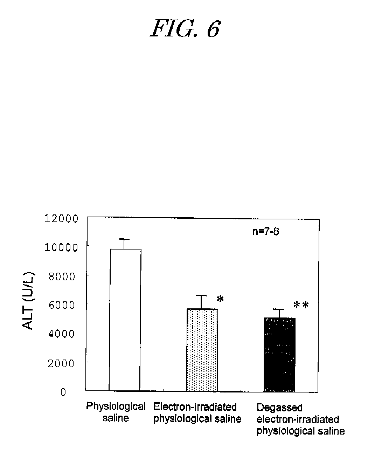

As shown in Fig. 6, when each one ml of the electron-irradiated physiological saline was administered intravenously 4 times at 0, 3, 10, and 20 minutes after reperfusion. The ALT value was 5,691±1,041 U/L. The ALT was inhibited by 41.9% in comparison with the group administered with the non-irradiated physiological saline of which ALT value was 9,793±911 U/L. This result is also statistically significant (t-test, p<0.05).

The inventors conducted a light microscope histopathological examination of the liver samples obtained respectively from the group without ischemic/reperfusion injury operation, the group administered with the physiological saline, and the group administered with the electron-irradiated physiological saline, after 3 hours from the reperfusion. As a result, in the group administered with the electron-irradiated physiological saline, alleviation of liver tissue injuries was observed. It is confirmed that this effect has an equivalent significance to the inhibitory effects on the increased ALT.

Here, Non-patent Document 1 describes that when hydrogen gas is given via inhalation to the animal model of liver ischemic/reperfusion injuries, the alleviation effect on injuries is observed. This posed a question that hydrogen gas is also be produced in the electron-irradiated physiological saline and this may contribute to alleviate the ischemic/reperfusion injuries. In order to investigate this, the following example was prepared.

The inventors separately prepared a hydrogen-dissolved physiological saline (1.2ppm of dissolved hydrogen level and minus 608mV of ORP) and investigated the inhibitory effect on ALT in the liver ischemic/reperfusion injuries model of rats in the same method shown in Fig. 6.

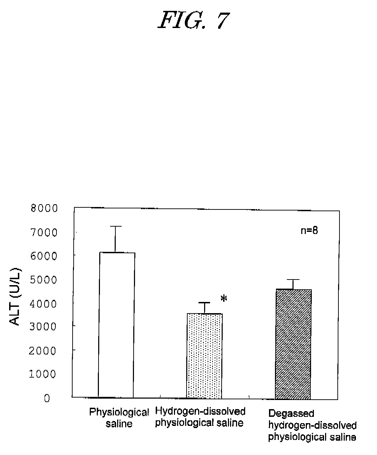

As shown in Fig. 7, in the group administered only with the physiological saline, the ALT value after 3 hours from the reperfusion was 6,133±1,058 U/L, while in the group administered with the hydrogen-dissolved physiological saline, the ALT value was 3,603±446 U/L. Therefore, the ALT value was inhibited by 41.2%, which shows a statistically significant difference (t-test, p<0.05).

The result is similar to the inhibitory effect on liver ischemic/reperfusion injuries by hydrogen gas inhalation. It is shown that the hydrogen gas serves as a radical scavenger, that is a substance eliminating active oxygen and free radicals originating from oxidized fatty acids, i.e., as anti-oxidative molecule.

Next, the inventors utilized the property that the hydrogen gas dissolved in a solution easily vaporizes, and prepared a degassed hydrogen-dissolved physiological saline by using a vacuum pump, and investigated again the inhibitory effect by using the above liver ischemic/reperfusion injuries model.

As shown in Fig. 7, in the group administered with the degassed hydrogen-dissolved physiological saline, the ALT value was decreased by 23.9% to 4, 670±341 U/L. Thus, by the degassing procedure, the statistically-significant inhibitory effect was no more observed (t-test, p<0.05).

Then, the inventors also prepared a degassed electron-irradiated physiological saline in the same manner, and investigated the effect by using the same liver ischemic/reperfusion injuries model of rats.

As shown in Fig. 6, in the group administered with the degassed electron-irradiated physiological saline, the ALT value was 5,098+764 U/L. The statistically-significant inhibitory effect of 47.9% was maintained (t-test, p<0.05), and was not largely decreased in comparison with the ALT inhibitory effect of 41.9% in the group administered with the non-degassed electron-irradiated physiological saline.

From this result, the electron-irradiated physiological saline stably maintains the anti-oxidative stress effect even after degassing. It becomes clear that the dissolved hydrogen gas is not an active substance for the anti-oxidative stress produced by electron irradiation in the physiological saline.

### (Second Embodiment)

There are difficulties in clinical application of the hydrogen-dissolved physiological saline, since stable preservation of the hydrogen gas is difficult and the solubility of the hydrogen gas is limited.

Here, the inventors found out that with regard to the stability of electron-irradiated physiological saline, the biological activity, that is, the reducing ability to inhibit the ischemic/reperfusion injuries, was no more effective when the solution was left at the room temperature for 24 hours, while the activity to inhibit the ischemic/reperfusion was maintained well for one hour after electron irradiation.

Under such condition, in order to use the electron-irradiated physiological saline in medical practice, it is necessary to supply the solution stably with the biological activity maintained. Therefore, the corona discharge method using the needle electrode in an environment exposed to the outside air, as shown in Fig. 1, is not suitable for this purpose. It is necessary to realize an apparatus for irradiating the physiological isotonic solution with electrons in an environment shielded from the outside air.

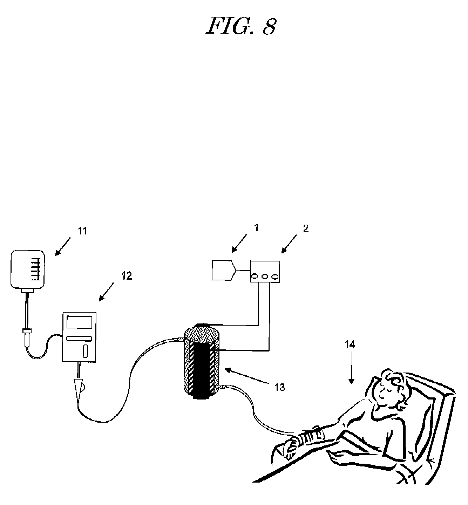

Moreover, it is necessary to prepare the electron irradiated solution when needed in clinical settings and continuously administer the solution. In order to achieve this, the inventors has devised, as shown in Fig. 8, an electron irradiation apparatus 13, which is connected with an infusion bag 11 filled with a physiological saline and an infusion pump 12. As similar to the first embodiment, the electron irradiation apparatus 13 is connected with the DC-type high voltage generator 2 which is connected to the DC power supply 1. The electron irradiation apparatus 13 is used to prepare an electron-irradiated physiological saline at bedside when needed and to intravenously administer the solution to a human body 14. An embodiment thereof will be shown below.

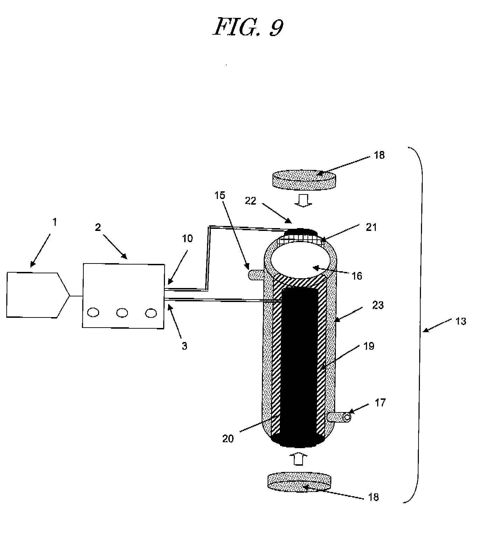

Fig. 9 shows one example of the column-type electron irradiation apparatus 13 shown in Fig. 8, which is suitable to continuously prepare the electron-irradiated physiological saline at bedside. The column-type electron irradiation apparatus 13 comprises a cylindrical column having a flow path 16 for a physiological saline inside thereof, and equipped with an inlet 15 and an outlet 17 for the physiological saline at the both ends thereof. Upper and lower openings of the column are respectively sealed with lids 18.

This column basically has a four-part configuration. For example, on a first part of the column, an electron discharging portion is formed by closely attaching a dielectric material 19 and a cathode electrode 20 having substantially the same size with each other. On a second part facing the electron discharging portion, an electron receiving portion is formed by closely attaching a conductive material pad 21 and an anode electrode 22 having substantially the same size with each other. Third and fourth parts, respectively positioned between the electrodes 20 and 22, are composed of insulation materials 23, 23 that insulates each of the electrodes, and those third and fourth parts are disposed to face each other.

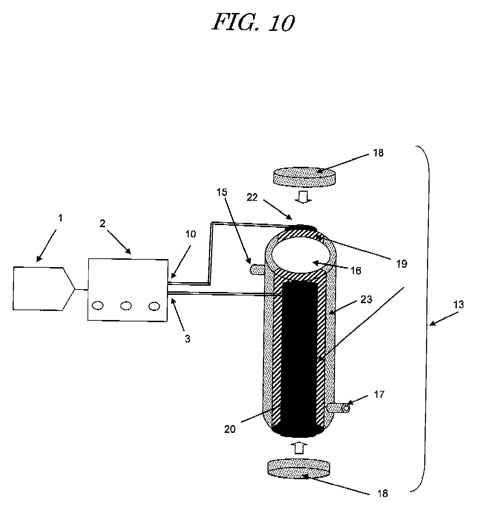

Fig. 10 shows a variation of the electron irradiation apparatus 13 shown in Fig. 9. In the electron irradiation apparatus 13 shown in Fig. 10, a dielectric material 19 is also disposed on the anode electrode 22 side, which is similar to the cathode electrode 20 side.

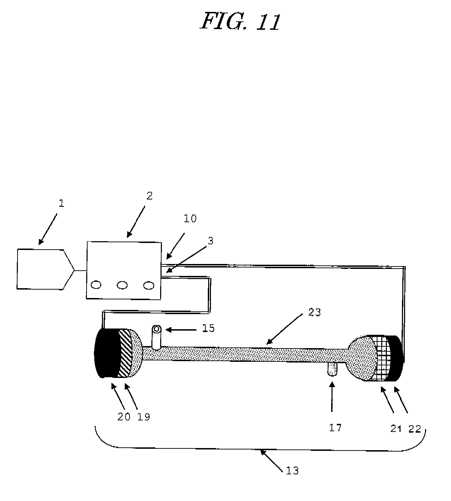

Fig. 11 shows another variation of the electron irradiation apparatus 13 shown in Fig. 9. The electron irradiation apparatus 13 of Fig. 11 is a column-type where the cathode electrode 20 attached with the dielectric material 19 and the anode electrode 22 attached with the conductive material pad 21 are disposed on the both ends of the column so that a long distance is achieved with respect to the electron flowing.

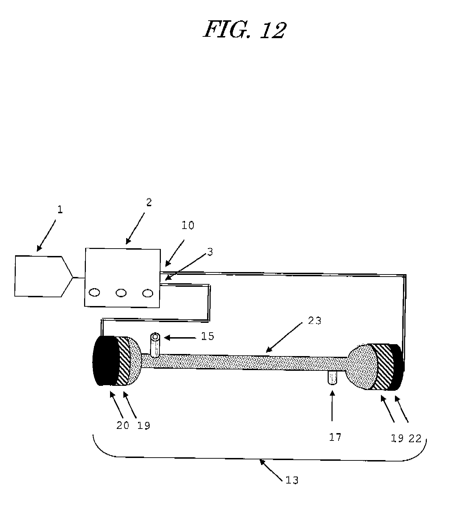

Fig. 12 shows another variation of the electron irradiation apparatus 13 shown in Fig. 10. The electron irradiation apparatus 13 shown in Fig. 12 is a column-type where the cathode electrode 20 attached with the dielectric material 19 and the anode electrode 22 attached with the dielectric material 19 are disposed on the both ends of the column so that a long distance is achieved with respect to the electron flowing.

As the dielectric material 19, for example, a carbon-containing dielectric material rubber with the nitrile rubber volume resistivity of 1.1E7Ω · CM and relative permittivity of 330 can be used, which was used in a DC-type dielectric barrier discharge electrode relating to the patent application previously filed by the inventors (International patent application number: PCT/JP2009/003714).

As for another example of the dielectric material 19, the dielectric material that has the volume resistivity of 8.6E13Ω·cm or lower as for insulation, and the relative permittivity of 5 or higher, will easily achieve the DC-type dielectric barrier discharge. As for materials thereof, for example, polyurethane, chloroprene rubber, and nitrile rubber can be used. Of course, the dielectric materials applied to the dielectric barrier discharge electrode is not limited to the above materials, and other materials having equivalent properties can also be used.

In order to precisely irradiate the physiological isotonic solution in each column shown in Figs. 9 and 10 with a certain amount of electrons from the surface of the dielectric material, the cathode electrode 20 is connected to the negative output terminal 3 of the high voltage generator 2 connected with the DC power supply 1 and the anode electrode 22 is connected to the positive output terminal 10 of the high voltage generator 2, thereby applying the high voltage to the electrodes.

Here, the discharge mechanism of the DC-type dielectric barrier discharge that is achieved by the presence of the said dielectric material 19 is described as follows. That is, when lots of minor discharges independent of each other occur in various spots on the surface of dielectric material 19, the applied voltage is a direct current. Therefore, once a discharge occurs, usually the electric potential on the surface spot of dielectric material is decreased and the discharge is suspended. However, it is explained that when volume resistivity and permittivity of the dielectric material 19 are selected appropriately as mentioned above, the dielectric material itself functions electrically as a circuit with distributed constants where a capacitor and a resistance are connected in parallel so as to continually develop minor discharges in various spots. As a result, the physiological saline flowing inside the flow path 16 of the column is continuously irradiated with electrons.

The DC-type power supply 1 is used in the above embodiment. However, in the case of the column which uses the dielectric materials 19 at both the electrodes as shown in Fig. 10, electron irradiation is also realized by using an AC power supply in place of a DC power supply.

In addition, the electron-irradiated physiological saline can be prepared in clinical practice when necessary, and administrated intravenously as an injection solution.

Furthermore, the inventors used columns having lengths different from the column shown in Fig. 11 (26mm in the inner diameter), and investigated the decreased value of ORP and the increased value of pH that are indicators of reducing ability of the physiological saline irradiated with electrons.

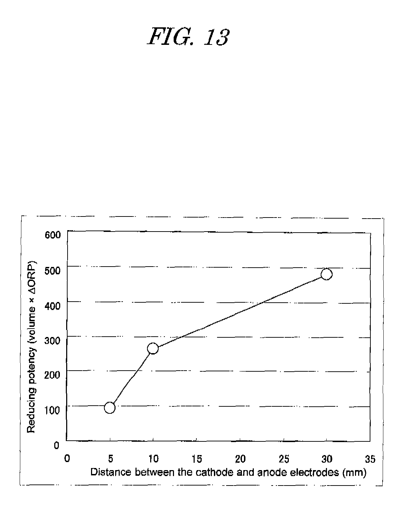

As the column length increased, proportional decrease of the ORP value and proportional increase of the pH value was observed. Specifically, as shown in Fig. 13, the reducing potency (volume × Δ ORP level) showed distinctive increases in proportion to the column length. Here, although the column became longer, an electric current (50 micro amperes) and a voltage (20V) were not changed. From this result, it is found that by appropriately increasing the column length, the electron irradiated water with a potent reducing ability can be prepared.

Especially in clinical application as an infusion solution, it is necessary to decrease the ORP value of the electron irradiated water within an appropriate time. By adjusting the distance between the electrodes of the electron irradiation apparatus integrated with the dielectric composite electrode, the appropriate reducing ability required for the electron irradiated water can be obtained within an appropriate time frame.

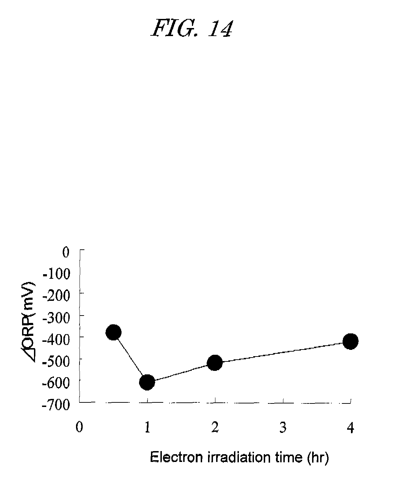

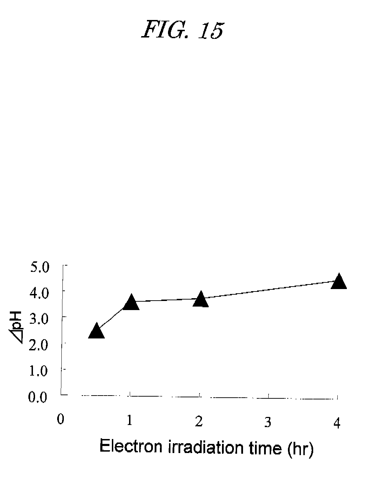

In addition, by application of 600 volts and 195 micro amperes when using the column integrated with the carbon-containing dielectric cathode electrode shown in Fig. 11 (volume: 5.8mL, dimension: 18mm in inner diameter and 23mm in length), the decreased value of the ORP was maximized at Δ608mV and the ΔpH was increased to 3.61 after 60 minutes of irradiation as shown in Figs. 14 and 15. Thus, in comparison to the indicators of reducing ability obtained in the electron irradiated water prepared by the corona discharge method for 60 minutes shown in Figs. 1, 2, and 3 (ΔORP 80, ΔpH 0.36), the electron irradiated water having the reducing ability 7.6-fold higher in the ORP level and 10-fold higher in the pH level can be prepared.

As shown in the above, by using the dielectric material at least for the cathode electrode, and by appropriately selecting the column length, the efficient method for preparing an electron irradiated water having reducibility can be achieved. Furthermore, this method can be applied to a medical emergency situation or to a continuously administered infusion.

### (Third Embodiment)

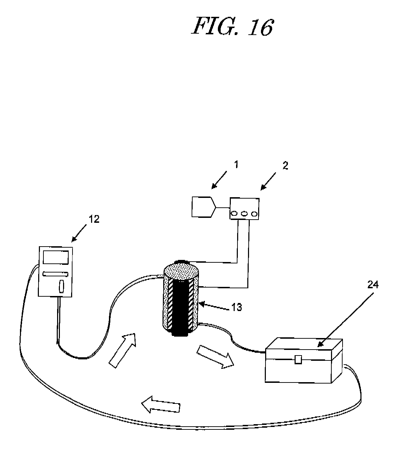

Third embodiment is, as shown in Fig. 16, an application to donor organ preservation in organ transplantation, and thus the human body 14 shown in Fig. 8 is replaced with an organ preservation container 24. The outlet of the column-type electron irradiation apparatus 13, filled with the organ preservation solution, is directly connected to an inlet of the organ preservation container 24. Then, from the outlet of the organ preservation container 24, via the infusion pump 12, the preservation solution is re-circulated to the inlet of the column-type electron irradiation apparatus 13. It is preferable that a refrigeration device using ice is provided on the outside of the organ preservation container 24.

In the third embodiment, while the preservation solution is circulated through the electron irradiation apparatus 13, the preservation solution can be continuously irradiated with electrons by the irradiation apparatus 13. Resultantly, as similar to the physiological saline shown in the first embodiment and the second embodiment, the preservation solution is stably supplied with the anti-oxidative stress effect. Accordingly, the organ inside the organ preservation container 24 can be preserved in a fresh state.

### (Fourth Embodiment)

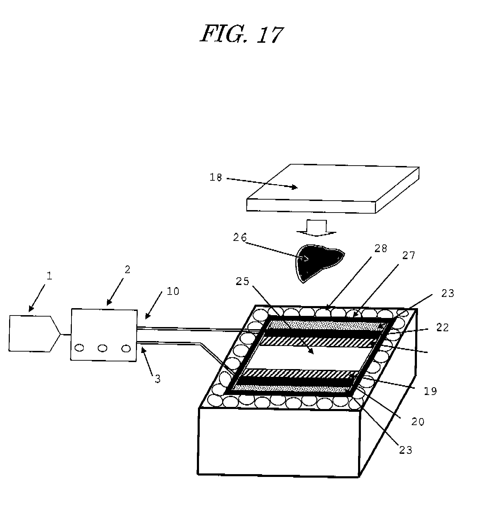

In a fourth embodiment, as shown in Fig. 17, the organ preservation container 24 is integrated in the electron irradiation column. In this embodiment, the volume of the column, which composes the electron irradiation apparatus, is enlarged for the purpose of the organ preservation so that the interior of the column itself can be used as the organ preservation container. The column for the electron irradiation is made to have an inner volume to sufficiently contain the organ preservation solution 25 and dimensions to store the donor organ 26. The whole column is covered by an insulator 27 and a refrigeration layer 28 using ice is equipped on the outside of the insulator so that the column is cooled while insulated.

In the fourth embodiment, the preservation solution can be supplied with the anti-oxidative stress effect as similar to the third embodiment, by irradiating the organ preservation solution 25 with electrons. Moreover, the whole size of the apparatus can be reduced by integrating the preservation container 24 into the electron irradiation apparatus 13.

## Claims

1. An electron-irradiated physiological isotonic solution, prepared by irradiating a physiological isotonic solution with electrons, wherein a decreased value of ORP is in a range of Δ30 mV to Δ608 mV or an increased value of hydrogen-ion concentration is in a range of Δ0.36 to Δ4.6.

2. The electron-irradiated physiological isotonic solution according to Claim 1, wherein the electron-irradiated physiological isotonic solution is an injection solution or an infusion solution for alleviating ischemic/reperfusion injuries.

3. An electron irradiation apparatus for preparing an electron-irradiated physiological isotonic solution, comprising:
a sealed column including a flow path for a physiological isotonic solution inside thereof, and also including an inlet and an outlet for the physiological isotonic solution communicating with the flow path at both ends thereof;
an electron discharging portion formed on a surface of the column by disposing a dielectric material and a cathode electrode closely-attached on an outside of the dielectric material;
an electron receiving portion formed on an opposite surface of the column to said surface provided with the cathode electrode by disposing a conductive material and an anode electrode closely attached on an outside of the conductive material,; and
a high voltage generator for applying a DC voltage between the electrodes equipped with a power supply thereof.

4. An electron irradiation apparatus for preparing an electron-irradiated physiological isotonic solution, comprising:
a sealed column including a flow path for a physiological isotonic solution inside thereof, and also including an inlet and an outlet for the physiological isotonic solution communicating with the flow path at both ends thereof,
wherein a dielectric material and a cathode electrode closely-attached on an outside of the dielectric material are disposed on a surface of the column,
a conductive material and an anode electrode closely-attached on an outside of the conductive material are disposed on an opposite surface of the column to said surface provided with the cathode electrode, and
a high voltage generator for applying a DC voltage or an AC voltage and a power supply thereof is equipped between the electrodes.

5. The electron irradiation apparatus for preparing the electron-irradiated physiological isotonic solution according to Claim 3 or 4, wherein a bag for supplying a physiological isotonic solution to be irradiated with electrons is connected to the inlet of the electron irradiation apparatus, and a tube for supplying the electron-irradiated physiological isotonic solution as an infusion solution is connected to the outlet of the electron irradiation apparatus.

6. An organ preservation apparatus equipped with the electron irradiation apparatus according to Claim 3 or 4,
wherein the organ preservation apparatus comprises an organ preservation container including an inlet and an outlet for an organ preservation solution and equipped with a refrigeration device of the organ preservation solution using ice, and the outlet and the inlet of the electron irradiation apparatus are respectively connected to the inlet and the outlet of the organ preservation container, and the organ preservation solution is circulated by a pump between the organ preservation container and the electron irradiation apparatus.

7. An organ preservation apparatus equipped in the electron irradiation apparatus according to Claim 3 or 4, wherein the flow path inside of the column is used as an organ preservation container.
